(19) Europäisches Patentamt / European Patent Office / Office européen des brevets

(11) **EP 4 609 786 A1**

(12) **EUROPÄISCHE PATENTANMELDUNG**

(43) Veröffentlichungstag:
03.09.2025 Patentblatt 2025/36

(21) Anmeldenummer: 25160571.3

(22) Anmeldetag: **27.02.2025**

(51) Internationale Patentklassifikation (IPC):
*A61B 5/083* (2006.01)   *A61B 5/087* (2006.01)
*A61B 5/091* (2006.01)   *A61B 5/097* (2006.01)
*A61M 16/08* (2006.01)   *A61M 16/00* (2006.01)
*A61M 16/10* (2006.01)

(52) Gemeinsame Patentklassifikation (CPC):
**A61B 5/0833; A61B 5/0836; A61B 5/087;
A61B 5/091; A61B 5/097; A61M 16/024;
A61M 16/0833; A61M 16/085; A61M 16/1005;
A61M 16/125;** A61M 2016/0036; A61M 2016/1025;
A61M 2016/103; A61M 2202/0208;
A61M 2205/3334;                      (Forts.)

(84) Benannte Vertragsstaaten:
**AL AT BE BG CH CY CZ DE DK EE ES FI FR GB
GR HR HU IE IS IT LI LT LU LV MC ME MK MT NL
NO PL PT RO RS SE SI SK SM TR**
Benannte Erstreckungsstaaten:
**BA**
Benannte Validierungsstaaten:
**GE KH MA MD TN**

(30) Priorität: **28.02.2024  DE 102024105703**

(71) Anmelder: **Löwenstein Medical Technology S.A.
2557 Luxembourg (LU)**

(72) Erfinder: **Kremeier, Peter
76149 Karlsruhe (DE)**

(74) Vertreter: **Löwenstein Medical IP
Löwenstein Medical Technology
GmbH + Co.KG IP Management
Kronsaalsweg 40
22525 Hamburg (DE)**

(54) **VERFAHREN UND VORRICHTUNG ZUR MESSUNG DER VOLUMETRISCHEN KAPNOMETRIE, OXYMETRIE UND DER FUNKTIONELLEN RESIDUALKAPAZITÄT (FRC)**

(57)  Die Erfindung betrifft ein Verfahren und eine Vorrichtung zur Messung von Stoffwechselparametern und der funktionellen Residualkapazität 14, das an ein Beatmungsgerät 15 angeschlossen ist. Zur Messung der CO2- und O2-Konzentration entnimmt die Vorrichtung eine Probe aus dem Atemkreislauf des Patienten und misst parallel dazu die Gasflussrate im Kreislauf des Patienten. Die Atemgasflussrate wird ohne Verzögerung gemessen. Die Daten zur Sauerstoff- und Kohlendioxidkonzentration unterscheiden sich zeitlich um die Transportverzögerung der Probenzufuhr vom Patientenkreislauf zu den CO2- und O2-Sensoren. Der Prozessor misst die Transportverzögerung T1 und T2 an charakteristischen Punkten und kompensiert sie. Zeitsynchronisierte CO2- und O2-Konzentrationsdaten werden zeitlich mit der Flussrate integriert und die volumetrischen Mengen von CO2, O2/N2 berechnet. Auf der Grundlage dieser Daten werden beispielsweise Stoffwechselparameter (REE, RQ) und/oder die volumetrische Kapnometriefunktion und/oder die funktionelle Residualkapazität (FRC) mit Hilfe der Stickstoffauswaschungsmethode berechnet. Die CO2-Daten werden zudem zur Erkennung von Artefakten und zum Ausschluss von Messfehlern sowie zum Ausschluss des CO2-Volumens aus dem ausgelaugten Stickstoff verwendet.

Fig. 3

EP 4 609 786 A1

(52) Gemeinsame Patentklassifikation (CPC): (Forts.)
A61M 2205/52; A61M 2230/40; A61M 2230/432;
A61M 2230/435; A61M 2230/437

**Beschreibung**

Die vorliegende Erfindung bezieht sich auf das Gebiet der Überwachung von Gasaustauschparametern, der Beatmungsmechanik von Beatmungsgeräten und der Optimierung der Steuerung von Beatmungsgeräten.

Die volumetrische Kapnometrie, die volumetrische Oxymetrie, die metabolischen Parameter und die funktionelle Residualkapazität (FRC) sind allesamt wichtige Aspekte der Atmungsphysiologie.

In einigen Patenten sind verschiedene Varianten einer Methode zur FRC-Messung mit einem Indikatorgas beschrieben, beispielsweise in den Dokumenten US 5540233 A, US 2022257141 A1, US 2002052560 A1. Als Indikatorgas werden in diesen Patenten Helium oder Schwefelhexafluorid oder Fluorpropan verwendet.

In der US 2002052560 A1 wird eine Methode zur Messung anhand von Apnoe-Episoden beschrieben. Bei dieser Messmethode ist das Indikatorgas das etCO2 des Patienten. Das Herzzeitvolumen wird zudem mit der Fick-Methode bestimmt.

Das Patent US8371298B2 offenbart eine Lösung mit folgenden Merkmalen:

- Der Sauerstoffsensor befindet sich in der Ausatemleitung des Atemkreislaufs;
- Verwendet wird ein langsam reagierender Sauerstoffsensor, um die durchschnittliche O2-Konzentration in der Ausatemleitung zu messen;
- Die CO2-Konzentration wird nicht berücksichtigt, was die Genauigkeit der Messungen verringert.

Die Aufgabe der vorliegenden Erfindung ist es, eine Vorrichtung zur Berechnung der volumetrischen Kapnometrie, volumetrischen Oxymetrie und der funktionellen Residualkapazität (FRC) anzugeben, die ohne zusätzliches Indikatorgas auskommt.

Einige fortschrittliche Beatmungsgeräte und Atemüberwachungssysteme können integrierte Messungen von Stoffwechselparametern und Lungenvolumen bieten. Diese Systeme weisen teilweise Vorrichtungen auf, die Echtzeitdaten zum Sauerstoffverbrauch, zur Kohlendioxidproduktion und zum Lungenvolumen liefern, ohne dass der Patient vom Beatmungsgerät getrennt werden muss.

Entscheidend ist hierbei jedoch die richtige Synchronisation der Messwerte, um Interferenzen zu vermeiden und die Ergebnisse plausibilisiert.

Die weitere Aufgabe der vorliegenden Erfindung ist es daher, die Messwerte der Gesamtgasdurchflussrate im Patientenkreislauf, des Sauerstoff- und des Kohlendioxidflusses über die Zeit zu synchronisieren.

Gelöst werden die genannten Aufgaben unter anderem durch eine Vorrichtung gemäß Anspruch 1, sowie durch ein Verfahren gemäß Anspruch 15.

Die Unteransprüche betreffen verschiedene voneinander unabhängige, vorteilhafte Weiterbildungen der vorliegenden Erfindung, deren Merkmale vom Fachmann im Rahmen des technisch Sinnvollen frei miteinander kombiniert werden können. Dies gilt insbesondere auch über die Grenzen der verschiedenen Anspruchskategorien hinaus.

Die Erfindung betrifft eine Vorrichtung zur zeitlich synchronisierten Messung von CO2- und O2-Anteilen im Atemgas und Atemgasfluss während der Beatmung mit einer Inspirationsleitung , einer Exspirationsleitung und einem Y-Verbinder und einer Leitung zum Patientenanschluss , wobei der Y-Verbinder die Inspirationsleitung , die Exspirationsleitung und die Leitung zum Patientenanschluss verbindet, aufweisend einen Fluss-Sensor zur Ermittlung von Strömungsdaten V(t), einen O2-Sensor zur Ermittlung von O2(t) und einen CO2-Sensor zur Ermittlung von CO2(t) und einen Prozessor dadurch gekennzeichnet, dass der Prozessor eingerichtet und ausgebildet ist,

- Strömungsdaten V(t) und CO2(t) im Speicher zu speichern,
- die Einatmungsphase IN oder die Ausatmungsphase EX im Verlauf von Messwerten des Strömungssensors und der Sensoren für CO2 und für O2 zu bestimmen,
- den zeitlichen Versatz zwischen den Messwerten des Strömungssensors und des CO2-Sensors gegenüber dem O2-Sensor zu bestimmen,
- die Messwerte entsprechend dem Zeitversatz zu synchronisieren.

Die Vorrichtung weist dazu einen Speicher auf oder der Speicher ist im Beatmungsgerät oder extern ausgebildet.

Die Vorrichtung erfasst Echtzeitinformationen über die biologisch wichtigsten Gase O2 und CO2 in der Atemluft. Dabei ist insbesondere die Ermittlung von CO2(t) über den CO2-Sensor hilfreich, um die Atemphase zu erkennen, da der CO2-Anteil im ausgeatmeten Gas in typischer Weise ansteigt. Auch können über die Ermittlung von CO2(t) mögliche Artefakte wie beispielsweise unerwünschte Leckagen entdeckt werden

Gemäß einer Ausführungsform ist der Prozessor 9 eingerichtet und ausgebildet, die synchronisierten Konzentrationsfunktionen O2(t), CO2(t) und Fluss V(t) zur Berechnung der volumetrischen Größen VO2 und VCO2 zu verwenden.

Nach einer vorteilhaften Ausführungsform ist der Prozessor 9 eingerichtet und ausgebildet, die VO2- und VCO2-Daten zur Berechnung der Stoffwechselparameter REE und RQ unter Verwendung der Weir-Gleichung zu verwenden. Die REE- und RQ-Parameter werden für jeden Atemzyklus berechnet und über ein vom Benutzer gewähltes Zeitintervall gemittelt.

Nach einer weiteren vorteilhaften Ausführungsform ist der Prozessor 9 eingerichtet und ausgebildet, die V(t)- und VCO2(t)-Daten zur Erstellung eines volumetrischen Kapnometriediagramms zu verwenden, wobei V(t) auf der vertikalen und VCO2(t) auf der horizontalen Achse liegt.

Vorteilhafterweise ist der Prozessor 9 eingerichtet und ausgebildet, die V(t)-, VO2(t)- und VCO2(t)-Daten zur Berechnung der funktionellen Rest-Lungenkapazität (FRC) zu verwenden.

Die Vorrichtung kann als Teil eines Beatmungsgerätes 15 ausgebildet sein oder ein zusätzliches Modul für ein Beatmungsgerät sein, wobei das Beatmungsgerät 15 dann einen Sauerstoffmischer 20 aufweist.

Der Prozessor 9 kann ein Teil der Vorrichtung oder ein Teil des Beatmungsgerätes 15 sein.

Der Prozessor 9 ist beispielsweise oder bevorzugt auch eingerichtet und ausgebildet, für eine FRC-Messung die Sauerstoffkonzentration im eingeatmeten Atemgas mittels des Sauerstoffmischers 20 periodisch um einen vorgegebenen Wert zu verändern, und durch

Messung der O2- und CO2-Menge in dem ausgeatmeten Atemgas eine Funktion der Stickstoffmenge in dem ausgeatmeten Atemgas zu ermitteln entsprechend der Formel

$$N2(t) = Vexp(t) - O2(t) - CO2(t);$$

wobei:

N2(t) - eine Funktion der Stickstoffmenge in der ausgeatmeten Luft;
Vexp(t) - eine Funktion der Ausatmungsflussrate;
O2(t) - eine Funktion der Sauerstoffmenge in der Ausatmungsluft;
CO2(t) - eine Funktion der Kohlendioxidmenge in der Ausatmungsluft.

In einer Ausführungsform ist der Prozessor 9 eingerichtet und ausgebildet, die FRC entsprechend folgender Formel zu bestimmen:

$$FRC = (VN1 - VN2)/(CN2 - CN1)$$

wobei

VN1 - Inspiratorisches Stickstoffvolumen
VN2 - Exspiratorisches Stickstoffvolumen
CN1 - Stickstoffvolumenanteil in der vorhergehenden Ausatmung
CN2 - Anteil des Stickstoffvolumens bei der aktuellen Ausatmung.

Nach einer weiteren Ausführungsform ist der Prozessor 9 eingerichtet und ausgebildet, die Berechnungen bei jedem Atemzyklus nach der Änderung der Sauerstoffkonzentration durchzuführen.

Nach einer weiteren vorteilhaften Ausführungsform sind der O2-Sensor und der CO2-Sensor über eine Probenahmeleitung mit dem Y-Verbindungsstück verbunden, um Atemgas zu den Sensoren zu führen.

Vorzugsweise weist die Vorrichtung eine Saugeinrichtung auf, die eingerichtet ist Atemgas über die Probenahmeleitung zu den Sensoren zu führen.

Weiter vorzugsweise ist der Fluss-Sensor in einer Leitung zwischen dem Y-Verbinder und dem Patienten angeordnet.

Damit ist von besonderem Vorteil, dass die Probenahmeleitung auf der einen Seite mit dem Y-Verbindungsstück und auf der anderen Seite über einen Probentrockner, welcher wässriges Kondensat aus dem analysierten Gas entfernt, verbunden ist und nachgeschaltet die Sensoren 7 und 8 angeordnet sind.

Die Erfindung betrifft auch ein Verfahren zur zeitlich synchronisierten Messung von CO2- und O2-Anteilen im Atemgas und Atemgasfluss während der Beatmung

mit einer Inspirationsleitung, einer Exspirationsleitung und einem Y-Verbinder und einer Leitung zum Patientenanschluss, wobei der Y-Verbinder die Inspirationsleitung, die Exspirationsleitung und die Leitung zum Patientenanschluss verbindet, aufweisend einen Fluss-Sensor 2, einen O2-Sensor 8 und einen CO2-Sensor 7 und einen Prozessor 9 dadurch gekennzeichnet, dass der Prozessor 9 eingerichtet und ausgebildet ist, aus dem Verlauf der Messwerte von dem Fluss-Sensor 2, von dem O2-Sensor 8 und dem CO2-Sensor 7 die Atemphase Inspiration IN oder Exspiration EX zu ermitteln und aus der jeweiligen Atemphase einen Zeitversatz der Messwerte von dem Fluss-Sensor 2, von dem O2-Sensor 8 und dem CO2-Sensor 7 zueinander zu bestimmen und die Messwerte entsprechend dem Zeitversatz zu synchronisieren.

Die Erfindung betrifft auch ein Verfahren und eine Vorrichtung zur Messung der volumetrischen Kapnometrie, Oxymetrie und der funktionellen Residualkapazität (FRC) und der Optimierung der Steuerung von Beatmungsgeräten.

Die Erfindung betrifft auch eine Vorrichtung zur zeitlich synchronisierten Messung von CO2- und O2-Anteilen im Atemgas und Atemgasfluss während der Beatmung:

- mit einer Inspirationsleitung, einer Exspirationsleitung und einem Y-Verbinder und einer Leitung zum Patientenanschluss, wobei der Y-Verbinder die Inspirationsleitung, die Exspirationsleitung und die Leitung zum Patientenanschluss verbindet,
- aufweisend einen Fluss-Sensor, einen O2-Sensor und einen CO2-Sensor und einen Prozessor dadurch gekennzeichnet, dass der Prozessor eingerichtet und ausgebildet ist,
- aus dem Verlauf der Messwerte von dem Fluss-Sensor, von dem O2-Sensor und dem CO2-Sensor die Atemphase Inspiration IN oder Exspiration EX zu ermitteln und aus der jeweiligen Atemphase einen Zeitversatz der Messwerte von dem Fluss-Sensor, von dem O2-Sensor und dem CO2-Sensor zueinander zu bestimmen und die Messwerte entsprechend dem Zeitversatz zu synchronisieren.

Die Messung von CO2 während der Beatmung ist wichtig, um die Atemfunktion und den Gasaustausch in den Lungen zu überwachen.

Die Hauptfunktion der Atmung besteht darin, Sauerstoff aufzunehmen und Kohlendioxid (CO2) abzugeben. Die CO2-Messung ermöglicht es, den Gasaustausch in den Lungen zu überwachen. Ein zu hoher oder zu niedriger CO2-Gehalt im Atemluftstrom kann auf Probleme im Gasaustausch hinweisen.

Die CO2-Messung gibt Aufschluss über die Effektivität der Ventilation, also darüber, wie gut die Luft in die Lungen gelangt und wie effizient das CO2 aus dem Körper entfernt wird. Eine unzureichende Ventilation kann zu einem Anstieg des CO2-Gehalts im Blut führen, was als Hyperkapnie bezeichnet wird.

Die Messung von CO2 ist besonders wichtig bei der Überwachung von Patienten, die mechanisch beatmet werden. Sie hilft dabei, Atemstörungen wie Hypoventilation oder Hyperventilation zu erkennen und entsprechende Anpassungen an der Beatmung vorzunehmen.

Bei Patienten, die sediert oder anästhesiert sind, kann die CO2-Messung dazu beitragen, die Tiefe der Sedierung zu überwachen und sicherzustellen, dass der Patient ausreichend beatmet wird.

Die Überwachung des CO2-Gehalts im Atemluftstrom kann dazu beitragen, Atemversagen frühzeitig zu erkennen. Dies ist besonders wichtig in kritischen medizinischen Situationen, in denen eine sofortige Intervention erforderlich sein kann.

Insgesamt ermöglicht die CO2-Messung während der Beatmung eine präzise Überwachung der Atemfunktion.

Insbesondere ist im Hinblick auf das Beatmungsgerät die Erkenntnis wesentlich, dass der von einem Atemzug zum nächsten Atemzug des künstlich beatmeten Patienten veränderliche Funktionszustand der beatmeten Lungen in die Regelung der Beatmungseinstellungen mit einbezogen wird. Durch die gleichzeitige Einbeziehung sowohl technischer als auch physiologischer Kenngrößen einerseits in Form der technischen Beatmungsparameter andererseits in Form der patientenspezifischen physiologischen Parameter, wie beispielsweise O2 und/oder CO2 und/oder FRC-Werte, in die Regelung des Beatmungsgerätes wird eine neue Dimension der Beatmungstherapie erreicht. Dieses Vorgehen ermöglicht eine sichere Steuerung der Beatmungstherapie.

Das vorschlagsgemäße Beatmungsgerät kann automatisiert den Zustand der Lunge über sich wiederholenden Messung von O2 und/oder CO2 und/oder FRC durchführen und in regelmäßigen Zeitabständen über die vorgeschlagene Steuerung automatisch die technischen Beatmungsparameter derart anpassen, dass eine erfolgreiche und auch schonende künstliche Beatmung des Patienten erfolgt.

Erfindungsgemäß findet die Lösung beispielsweise Anwendung in einem Beatmungsgerät mit mit offenem oder geschlossenem Regelkreis und Rückkopplungsregelungsalgorithmen, um die Einstellungen des Beatmungsgeräts automatisch auf der Grundlage der physiologischen Messwerte anzupassen.

Das Beatmungsgerät zielt darauf ab, die Beatmungs- und Gasaustauschparameter zu optimieren, um die Ergebnisse für den Patienten zu verbessern und das Risiko einer beatmungsinduzierten Lungenschädigung zu verringern.

Die volumetrische Kapnometrie, die voluemtrische Oxymetrie, die metabolischen Parameter und die funktionelle Residualkapazität (FRC) sind allesamt wichtige Aspekte der Atmungsphysiologie. Im Folgenden wird dargestellt wie jeder dieser Parameter erfindungsgemäß oder abgewandelt berechnet werden kann und genutzt werden kann.

Die volumetrische Kapnometrie ist eine Technik zur Messung des Volumens des von der Lunge ausgeatmeten Kohlendioxids (CO2) im Laufe der Zeit.

Die wichtigsten Parameter der volumetrischen Kapnometrie sind das Kapnogramm und das volumetrische Kapnogramm, eine grafische Darstellung der CO2-Konzentration und -Menge im zeitlichen Verlauf des Atemzyklus.

Die Fläche unter der volumetrischen Kapnogrammkurve kann Aufschluss über die Gesamtmenge des ausgeatmeten CO2 geben, die mit der Effizienz der Ventilation zusammenhängt. Die $CO_2$-Konzentration in der Ausatemluft ist der wichtigste Parameter für die Überwachung der Beatmungstherapie.

Die Fläche unter der volumetrischen Kapnogrammkurve wird in der Regel mit spezieller Software oder Ausrüstung gemessen.

Diese Fläche stellt das Gesamtvolumen des während des Atemzyklus ausgeatmeten Kohlendioxids (CO2) dar.

Die Integration der Kapnogrammkurve ist bei Kapnographiegeräten häufig automatisiert.

Weiter vorzugsweise kann die Messung von Kohlendioxidwerten mittels der volumetrischen Kapnographie erfolgen und unmittelbar den CO2-Gasaustausch in der Lunge des Patienten repräsentierende Parameter, beispielsweise end-exspiratorischer CO2-Partialdruck im ausgeatmeten Gasgemisch, alveolärer CO2-Partialdruck, oder bei einem einzelnen Atemzug des Patienten eliminiertes Volumen an CO2, bestimmen.

Die Kapnographie ist daher ein geeignetes Mittel, um die Menge bzw. den Anteil des exspiratorischen Kohlenstoffdioxids, kurz exspiratorischer CO2 genannt, zu bestimmen und auch graphisch darzustellen. Dabei wird die CO2-Kinetik maschinell beatmeter Patienten auf nichtinvasive Art und in Echtzeit dargestellt. Insbesondere die volumetrische Kapnographie stellt ein geeignetes Mittel für das klinische Monitoring maschinell beatmeter Patienten dar.

Mittels der volumetrischen Kapnographie kann dabei die CO2-Konzentration in Atemgasen während des Atemzyklus gemessen werden. Die CO2-Konzentration wird beispielsweise durch die Absorption von Infrarotlicht gemäß dem Lambert-Beer'schen Gesetz berechnet und üblicherweise als Partialdruck in der Einheit mmHg ausgedrückt. Die graphische Darstellung der CO2-Elimination während der Atmung wird als Kapnogramm bezeichnet und das entsprechende Messgerät als Kapnograph.

Sogenannte Nebenstrom-Kapnographen sind Vorrichtungen, die eine Atemgasprobe an der Atemwegsöffnung von dem beatmeten Patienten absaugen, über ein Schlauchsystem zu entfernt von der Ansaugstelle gelegen Sensoren transportieren, um dort das darin enthaltene CO2 zu messen. Das Nebenstromverfahren kann auch bei nicht-intubierten Patientinnen und Patienten über eine Nasenbrille mit $CO_2$-Absaugleitung zum Einsatz kommen. Außerdem bietet das Nebenstromverfahren den großen Vorteil, dass keine zusätzliche Messküvette notwendig ist. Dadurch ist der Totraum des Patientenschlauchsystems deutlich verringert und auch das Gewicht des Beatmungsschlauches am Patientenende ist kleiner. Die $CO_2$-Messung erfolgt beim Nebenstromverfahren mit einer leichten zeitlichen Verzögerung und ist dadurch etwas langsamer als die Hauptstrommessung.

Hauptstrom-Kapnographen hingegen sind Geräte, bei denen sich die CO2-Sensoren und in manchen Fällen auch die Flusssensoren in einem Messkopf am Y-Stück des Beatmungsschlauchs befinden, sodass mit dieser Methode in situ Messungen nahe der Atemwegsöffnung durchgeführt werden können. Hauptstrom-Kapnographen verursachen keine Volumenverluste und messen das gesamte Atemgasvolumen. Durch die zusätzliche Messküvette zwischen Patientenventil und Tubus kommt es beim Hauptstromverfahren zu einem erhöhten Totraumvolumen.

Bei der Messung tritt keine zeitliche Verzögerung auf und es kann die gesamte Luftmenge berücksichtigt werden. Sowohl Nebenstrom-Kapnographen als auch Hauptstrom-Kapnographen können im Rahmen der vorliegenden Erfindung Verwendung finden, wobei die genannten Nebenstrom-Kapnographen bevorzugt genutzt werden. Die Kapnographie beschreibt die kontinuierliche Messung des endtidalen CO2 (etCO2) im Ausatemgas, die über Infrarotspektroskopie im Seitenstromverfahren erfolgt.

Die Kapnographie wird in der Regel gemäß ihrer graphischen Darstellung klassifiziert, wobei die zeitbasierte oder Standardkapnographie die am meisten verbreitete Art von Kapnogrammen ist. Hierbei wird die CO2-Konzentration im zeitlichen Verlauf geplottet. Die volumenbasierte oder volumetrische Kapnographie hingegen stellt die Menge des bei einem Atemzug eliminierten Kohlendioxids über dem ausgeatmeten Volumen des Atemzuges dar. Im Unterschied zur Standardkapnographie kann die volumetrische Kapnographie in vorteilhafter Weise volumetrische Parameter erfassen, die von klinischer Bedeutung sind. Dazu zählen die pulmonale Elimination von CO2, der Totraum sowie die alveoläre Belüftung. Sowohl die zeitbasierte oder Standardkapnographie als auch die volumenbasierte oder volumetrische Kapnographie können im Rahmen der vorliegenden Erfindung Verwendung finden.

In analoger Weise zu dem zuvor diskutierten unmittelbar den CO2-Gasaustausch in der Lunge des Patienten repräsentierenden können alternativ oder ergänzend auch in vorteilhafter Weise unmittelbar den Sauerstoff-Gasaustausch (kurz: O2-Gasaustausch) in der Lunge des Patienten repräsentierenden Parameter als patientenspezifischer physiologischer Parameter eingesetzt werden.

Nach einem weiteren Aspekt der Lehre wird ein Beatmungsgerät zur künstlichen Beatmung eines Patienten vorgeschlagen. Das vorschlagsgemäße Beatmungsgerät umfasst: eine Steuereinrichtung, die zum Festlegen wenigstens eines technischen Beatmungsparameters eingerichtet ist, wobei auf Grundlage des technischen Beatmungsparameters das Beatmen des Patienten erfolgt, wobei der wenigstens eine technische Beatmungsparameter wenigstens einem der Beatmungparameter Atemminutenvolumen, Tidalvolumen, Atemfrequenz, positiver end-exspiratorischer Druck, oder durch das Beatmungsgerät bereitgestellte, inspiratorische Sauerstoffkonzentration entspricht; sowie eine Regelungseinheit, die in Kommunikation mit der Messeinrichtung und mit der Steuereinrichtung ist. Die Steuereinheit ist derart eingerichtet, dass sie eine Anpassung des wenigstens eines technischen Beatmungsparameters auf Grundlage der sich wiederholenden Messung von O2 und/oder CO2 und/oder FRC durchführt.

Zur Durchführung der volumetrischen Oxygraphie sollten schnell reagierende Sauerstoffsensoren verwendet werden, deren Leistung in der gleichen Größenordnung wie die des Kapnographen liegen. Andernfalls kommt es zu Messfehlern aufgrund einer Verzerrung der Form der volumetrischen Oxygraphie.

Die O2- und CO2-Konzentrationen sollten nacheinander in derselben Gasprobe gemessen werden.

Metabolische Parameter

Stoffwechselparameter im Zusammenhang mit der Atmungsphysiologie beziehen sich häufig auf Parameter, die sich auf den Sauerstoffverbrauch (VO2) und die Kohlendioxidproduktion (VCO2) beziehen.

Der Respiratorische Quotient (RQ) ist ein solcher Stoffwechselparameter und wird als das Verhältnis von VCO2 zu VO2 berechnet. RQ = VCO2 / VO2.

Der RQ gibt Aufschluss über das Substrat, das verstoffwechselt wird. So deuten beispielsweise RQ-Werte von 1,0 auf einen Kohlenhydratstoffwechsel hin, während Werte unter 1,0 auf einen Fettstoffwechsel hindeuten.

Bei der Messung wird die ein- und ausgeatmete Luft auf ihre Sauerstoff- und Kohlendioxidkonzentration hin untersucht.

Ensprechende Vorrichtungen berechnen VO2 und VCO2 automatisch auf der Grundlage der Gaskonzentrationen und des Luftstroms.

Diese Technik liefert wertvolle Informationen über den Stoffwechsel und den Gasaustausch der Atemwege des Patienten.

Funktionelle Residualkapazität (FRC):
FRC ist das Luftvolumen, das sich am Ende der passiven Ausatmung in der Lunge befindet. Sie stellt das Gleichgewicht zwischen dem elastischen Rückstoß der Lunge und dem Rückstoß der Brustwand nach außen dar.

Die FRC kann mit verschiedenen Techniken gemessen werden, darunter Body-Plethysmographie, Heliumverdünnung oder Stickstoffauswaschung.

Bei dieser Technik wird die Luft in der Lunge durch eine bekannte Konzentration von Stickstoff ersetzt.

Das anfängliche Lungenvolumen (V1) wird gemessen, bevor der Stickstoff eingeführt wird. Die Stickstoffkonzentration (F1) und die Endkonzentration des Stickstoffs (Fi) werden gemessen, sobald ein Gleichgewicht erreicht ist.

Anschließend wird das Endvolumen der Lunge (V2) gemessen.

Eine Formel für die Berechnung der FRC mit der Stickstoffauswaschungstechnik lautet FRC = V1 + (F1/Fi) * (V2 - V1), wobei V1 das Anfangsvolumen der Lunge, F1 die Anfangskonzentration des Stickstoffs, Fi die Endkonzentration des Stickstoffs und V2 das Endvolumen der Lunge ist.

Gemäß einer ersten vorteilhaften Ausführungsform des Ventilators ist vorgesehen, dass der Ventilator umfasst:

- einen Gasströmungssensor, der am Y-Verbindungsstück angeordnet ist,
- einen Seitenstrom-Kapnographen,
- ein Oximeter für den seitlichen Fluss.

Gemäß einer zweiten vorteilhaften Ausführungsform des Beatmungsgeräts ist vorgesehen, dass das Beatmungsgerät Folgendes umfasst:

- einen Seitenstromkapnographen
- ein Seitenstrom-Oximeter.

Die Vorrichtung verfügt nicht über einen Gasflusssensor am Y-Anschluss, sondern verwendet die von den integrierten Flusssensoren erhaltenen Flussdaten.

Die Erfindung betrifft ein Verfahren und eine Vorrichtung 14 zur Messung von Stoffwechselparametern und der funktionellen Residualkapazität. Zur Messung der CO2- und O2-Konzentration entnimmt die Vorrichtung eine Probe aus dem Atemkreislauf des Patienten und misst parallel dazu die Gasflussrate im Kreislauf des Patienten. Die Atemgasflussrate wird ohne Verzögerung gemessen. Die Daten zur Sauerstoff- und Kohlendioxidkonzentration unterscheiden sich zeitlich um die Transportverzögerung der Probenzufuhr vom Patientenkreislauf zu den CO2- und O2-Sensoren. Der Prozessor misst die Transportverzögerung T1 und T2 an charakteristischen Punkten und kompensiert sie. Zeitsynchronisierte CO2- und O2-Konzentrationsdaten werden zeitlich mit der Flussrate integriert und die volumetrischen Mengen von CO2, O2/N2 berechnet. Auf der Grundlage dieser Daten werden beispielsweise Stoffwechselparameter (REE, RQ) und/oder die volumetrische Kapnometriefunktion und/oder die funktionelle Residualkapazität (FRC) mit Hilfe der Stickstoffauswaschungsmethode berechnet. Die CO2-Daten werden zudem zur Erkennung von Artefakten und zum Ausschluss von Messfehlern sowie zum Ausschluss des CO2-Volumens aus dem ausgelaugten Stickstoff verwendet.

Die Vorrichtung für volumetrische Kapnometrie, Stoffwechselparameter und funktionelle Residualkapazität (im Folgenden als Vorrichtung bezeichnet) ist für die Verwendung in Verbindung mit Beatmungsgeräten oder als integrierter Bestandteil von Beatmungsgeräten vorgesehen. Die Vorrichtung verfügt über einen Prozessor, der die Parameter der volumetrischen Kapnometrie, des Stoffwechsels und den Wert der funktionellen Residualkapazität berechnet. Diese Daten werden beispielsweise zur Anzeige an das Beatmungsgerät übertragen.

Gemäß der Erfindung werden verschiedene Parameter des Gasaustauschs in der Lunge des Patienten, Stoffwechselparameter und die Atmungsmechanik bestimmt, die normalerweise von mehreren Geräten gemessen werden, sowie zuverlässigen Algorithmen für die Messung dieser Parameter bereitgestellt.

Figur 1 eine schematische Darstellung von Vorrichtung und Beatmungsgerät
Figur 2 eine detaillierte, schematische Darstellung der Vorrichtung
Figur 3 drei Diagramme, die zeitbasierte Verläufe von Fluss, Sauerstoff- und KohlendioxidWerten zeigen

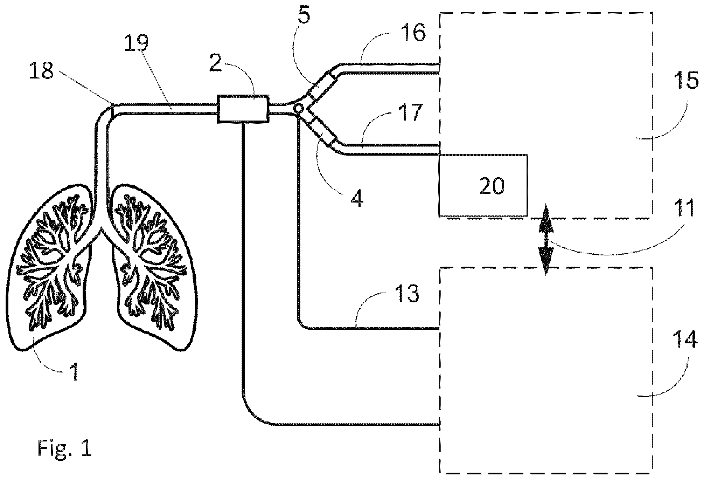

Die Fig.1 zeigt eine schematische Übersicht des Geräts bestehend aus der Vorrichtung und dem Beatmungsgerät. Die Vorrichtung 14 und das Beatmungsgerät 15 bilden ein komplettes System, das mit dem Patienten 1 verbunden ist. Der Atemkreislauf des Beatmungsgeräts 15 besteht aus einer Inspirationsleitung 17, einer Exspirationsleitung 16 und einem Y-Verbindungsstück 3. Der Atemkreislauf des Beatmungsgeräts ist über den Y-Anschluss 3 mit dem Patienten 1 verbunden.

Y-Verbindungsstück 3. Während der Inspirationsphase wird der Lunge des Patienten 1 über die Inspirationsleitung 17 und den Anschluss 4 des Y-Verbindungsstücks frisches Gas zugeführt. Während der Exspirationsphase verlässt das Gas die Lunge des Patienten 1 über den Anschluss 5 des Y-Verbinders, die Ausatmungsleitung 16 und das (nicht gezeigte) Ausatmungsventil des Beatmungsgeräts in die Umgebung. Je nach Beatmungsmodus kann ein Teil des Inspirationsflusses direkt von der Inspirationsleitung in die Exspirationsleitung geleitet werden, um den für die Funktion des Inspirationstriggers erforderlichen Unterstützungsfluss zu gewährleisten.

Die Vorrichtung 14 ist mit dem Atemkreislauf des Beatmungsgeräts (Y-Verbindung 3) und über die Ausgangsschnittstelle 11 mit dem Steuersystem des Beatmungsgeräts verbunden, wie in FIG. 1 dargestellt.

Der Fluss-Sensor 2 der Vorrichtung ist zwischen dem Y-Verbinder 3 und dem Patienten 1 angeschlossen. Die Probenahmeleitung 13 der Vorrichtung 14 ist auf der einen Seite mit dem Y-Verbindungsstück 3 und auf der anderen Seite über einen Probentrockner 12, welcher wässriges Kondensat aus dem analysierten Gas zieht, mit dem Vorrichtung 14 verbunden. Wenn das Beatmungsgerät über einen Durchflusssensor verfügt, ist der Sensor 2 nicht angeschlossen und die Durchflussdaten werden vom Beatmungsgerät über die Ausgangsschnittstelle 11 an die Vorrichtung 14 übertragen.

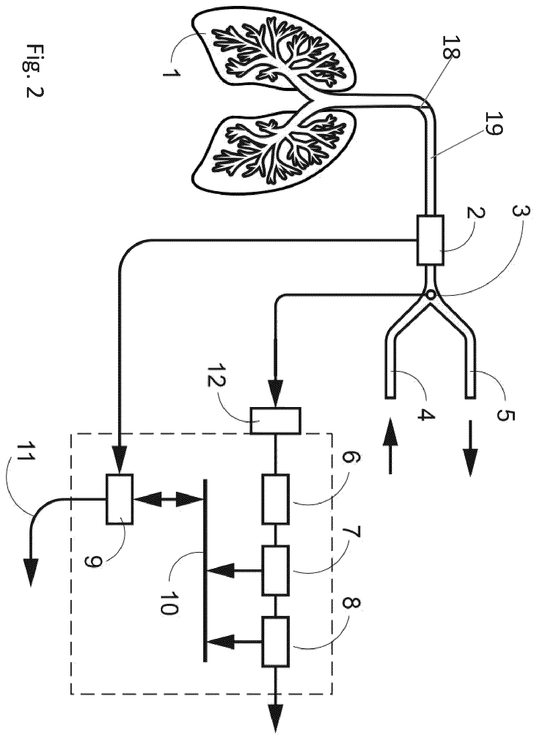

Fig.2 ist bietet eine schematische Grundlage für die folgende Beschreibung der Funktionsweise der Vorrichtung.

Der Flusssensor 2 misst den Gasdurchfluss während der Inspirations- und Exspirationsphase. Die Daten der Durchflussrate während der Inspirationsphase werden integriert und das Volumen wird berechnet, ebenso wird das Exspirationsvolumen berechnet. Wenn das inspiratorische und das exspiratorische Volumen unterschiedlich sind, wird die Leckagerate berechnet.

Die Probenahmeleitung 13 der Vorrichtung 14 ist mit der Probenentfeuchtungsvorrichtung 12 verbunden. Die Probe wird mit Hilfe des Mikrokompressors 6 aus dem Patientenkreislauf angesaugt. Die Probe durchläuft den Probenentfeuchter 12, und das entwässerte Gas wird an den Kohlendioxidkonzentrationssensor 7 (Kapnograph) und weiter an den Sauerstoffkonzentrationssensor 8 weitergeleitet.

Die Gasprobe durchläuft die Probenahmeleitung 13 in einer Zeit von beispielsweise 0,2 - 3 Sekunden, je nach Länge der Probenahmeleitung. Während dieser Transportzeit bleiben die CO2-Konzentrationsdaten des Sensors 7 hinter den Daten der Durchflussrate zurück. Die O2-Daten des Sensors 8 werden zeitlich später als die Durchflussdaten des Sensors 2 als auch zeitlich später als die CO2-Daten des Sensors 7 ermittelt, da der O2-Sensor 8 dem CO2-Sensor 7 nachgeschaltet ist.

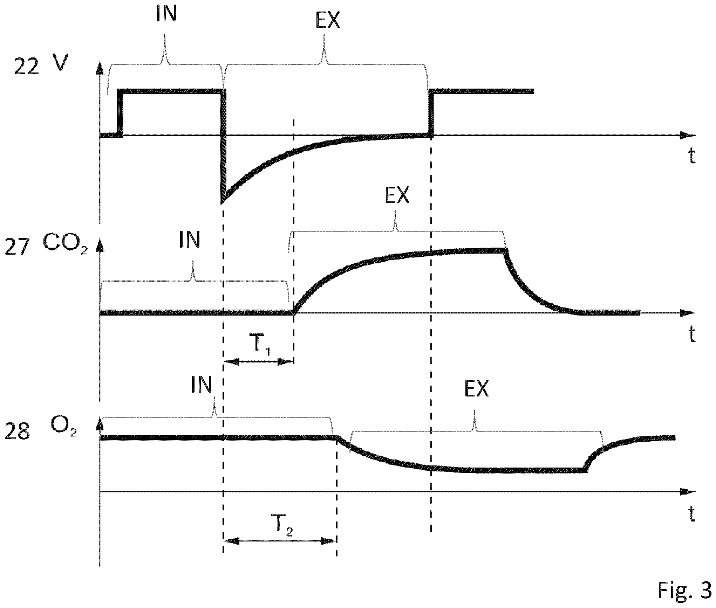

Die Fig.3 zeigt einen Verlauf der Messwerte Fluss, Sauerstoff und Kohlendioxid über die Zeit während der Beatmung eines Patienten. Figur 3 zeigt in der Spur 22 die Daten des Flusssensors 2. Diese werden integriert, um das Volumen V zu ermitteln. Aus dem Volumenverlauf erkennt man abwechselnde Phasen der Inspiration In und der Exspiration Ex im Zeitverlauf t.

In der Spur 27 erkennt man die Daten des CO2-Sensors 7. Aus dem Verlauf erkennt man abwechselnde Phasen der Inspiration In und der Exspiration Ex im Zeitverlauf t. Im Vergleich mit den Zeitpunkten der Inspiration In und der Exspiration Ex aus der Spur 22, erkennt man einen zeitlichen Versatz T1, der sich auf den Beginn der Exspiration Ex bezieht.

In der Spur 28 erkennt man die Daten des O2-Sensors 8. Aus dem Verlauf erkennt man abwechselnde Phasen der Inspiration In und der Exspiration Ex im Zeitverlauf t. Im Vergleich mit den Zeitpunkten der Inspiration In und der Exspiration Ex aus der Spur 22, erkennt man einen zeitlichen Versatz T2, der sich auf den Beginn der Exspiration Ex bezieht.

Der zeitliche Versatz ergibt sich aus dem verzögerten Transport des Gases vom Y-Stück 3 über die Probenahmeleitung 13 zu der Vorrichtung 14. Wegen der Transportverzögerung liegen die Daten der CO2- und O2-Konzentration später vor als die Flussdaten. Wegen der Transportverzögerung können die Daten der CO2- und O2-Konzentration und des Durchflusses V nicht zusammen verarbeitet werden, ohne sie zeitlich zu synchronisieren. Zur Synchronisierung müssen die Werte der Verzögerungen T1 und T2 bestimmt werden.

Dazu werden die charakteristischen Punkte des Beginns und des Endes von Inspiration und Exspiration auf den Kurven 27, 28 der CO2- und O2 -Konzentration bestimmt. Die Bestimmung erfolgt bevorzugt automatisiert anhand von hinterlegten Algorithmen, die aus dem Verlauf der Kurven die Atemphase erkennen. Die Bestimmung basiert auch auf der Erkenntnis, dass in der Exspiration der CO2 Anteil im Atemgas erhöht ist und der O2-Anteil vermindert ist.

Dann wird die Flusskurve 22 um den Wert von T2 und die CO2-Konzentrationskurve 27 um den Wert von T2-T1 verzögert, wodurch die zeitliche Synchronisierung aller Kurven erreicht wird.

In diesem Ausführungsbeispiel sind die Sensoren in Strömungsrichtung des Gases nacheinander angeordnet; zuerst der CO2-Sensor und nachfolgend dann O2-Sensor. Der O2-Sensor ist der zeitlich am weitesten zurückliegende. Daher sollte das Durchflusssignal um die Zeit T2 verzögert werden und das CO2-Signal um eine kürzere Zeit T2-T1.

Die synchronisierten Signale der CO2- und O2-Sensoren und der Strömungsgeschwindigkeit V werden an den Prozessor 9 (siehe Fig. 2) übertragen, wo die Parameter auf der Grundlage von Arbeitsalgorithmen berechnet und über die externe Schnittstelle 11 beispielsweise an das Beatmungsgerät zur Anzeige auf dem Bildschirm übertragen werden können.

Die Vorrichtung verfügt zudem über Algorithmen zur Bestimmung der funktionellen Residualkapazität (FRC), der metabolischen Parameter und der volumetrischen Kapnometrie.

Der Ablauf beispielhafter Schritte für die Ermittlung der FRC und die synchronisierten Konzentrationsfunktionen O2(t), CO2(t) und Fluss V(t) ist wie folgt:
Die Vorrichtung kann an den Datenbus des Beatmungsgerätes angeschlossen werden oder sie ist ein Bestandteil des Beatmungsgerätes.

Die Vorrichtung ist auch mit dem Atemkreislauf des Beatmungsgerätes am Y-Anschluss verbunden, um eine Probe zur Analyse zu entnehmen.

Die Vorrichtung nutzt den Flow-Sensor, der im Bereich des Y-Anschlusses mit dem Atemkreislauf des Beatmungsgerätes verbunden ist.

Die Vorrichtung selbst weist keinen Flow-Sensor auf und kann Flow-Daten vom Beatmungsgerät verwenden, die beispielsweise über den Datenbus übertragen werden.

Die Vorrichtung selbst verfügt beispielsweise nicht über einen Durchflusssensor und kann Durchflussdaten verwenden, die beispielsweise von einem Beatmungsgerät empfangen und welche an die Vorrichtung übertragen werden.

Die Vorrichtung entnimmt eine Gasprobe zur Messung der Sauerstoffkonzentration O2 und der Kohlendioxidkonzentration CO2.

Die Vorrichtung verfügt über einen Zeitsynchronisationsalgorithmus, der die Verzögerung der Gasprobe mit Bezug auf die Strömungsgeschwindigkeitsfunktion V(t) kompensiert.

Die synchronisierten Konzentrationsfunktionen O2(t), CO2(t) und Fluss V(t) werden zur Berechnung der volumetrischen Größen VO2 und VCO2 verwendet.

Die VO2- und VCO2-Daten werden optional auch zur Berechnung der Stoffwechselparameter REE und RQ unter Verwendung der Weir-Gleichung verwendet.

Die V(t)- und VCO2(t)-Daten werden zur Erstellung eines volumetrischen Kapnometriediagramms verwendet, wobei V(t) auf der vertikalen und VCO2(t) auf der horizontalen Achse liegt.

Die V(t)-, VO2(t)- und VCO2(t)-Daten werden zur Berechnung der funktionellen Rest-Lungenkapazität (FRC) verwendet.

Die berechneten Parameter werden über beispielsweise den Datenbus des Beatmungsgerätes ausgegeben oder an das Beatmungsgerät übertragen und können dort angezeigt und/oder gespeichert oder zusätzlich für die Regelung der Beatmung genutzt werden.

Der Patient wird mit einem Beatmungsgerät beatmet, das über einen Y-Konnektor mit dem Patienten verbunden ist. Der Y-Konnektor ist mit einer Inspirationsleitung für frisches Atemgas, das vom Beatmungsgerät zugeführt wird, und einer Exspirationsleitung zum Abführen des ausgeatmeten Gases aus der Lunge des Patienten verbunden.

Das frische Atemgas ist eine Mischung aus Luftfraktionen, Sauerstoff und Wasserdampf. Das ausgeatmete Gas besteht aus Kohlendioxid-, Luft-, Sauerstoff- und Wasserdampffraktionen.

Die Gasentnahme für die betreffende Vorrichtung zur Messung der Gaskonzentrationen erfolgt am Y-Verbindungsstück. An diesem Punkt wird auch die Gasdurchflussrate gemessen. So kann nicht nur die Konzentration, sondern auch die Menge an Sauerstoff und Kohlendioxid bei der Ein- und Ausatmung berechnet werden. Wird angenommen, dass die Wasserdampfkonzentration bei Inspiration und Exspiration gleich ist, so ist der Restgasanteil Stickstoff.

Durch die Berechnung der Gasmenge im Bereich des Y-Verbindungsstücks 3 werden Fehler aufgrund des Unterstützungsflusses und möglicher Gaslecks im Kreislauf eliminiert. Der Unterstützungsfluss im Beatmungsgerät wird normalerweise zur Betätigung des inspiratorischen Triggers benötigt und fließt direkt von der Inspirationsleitung zur Exspirationsleitung.

Die Platzierung des Flow-Sensors in der Nähe des Y-Verbinders ermöglicht es, die Leckage zu erkennen und die Größe der Leckage zu schätzen und die notwendigen Anpassungen der Berechnungsergebnisse vorzunehmen. Oder, im Falle einer großen Leckage, eine Warnung auszugeben, dass die Berechnungsergebnisse unzuverlässig sind. Durch die Kohlendioxidmessung werden auch Fehler bei der FRC-Schätzung aufgrund des Vorhandenseins von CO2 im Atemkreislauf vermieden.

Um Fehler zu reduzieren, müssen die dynamischen Eigenschaften der Sauerstoff- und Kohlendioxidsensoren hoch genug sein, um die Form der inspiratorischen und exspiratorischen Gasfraktionsströme nicht zu verzerren.

Die Messung der Kohlendioxidkonzentration beim Ausatmen ermöglicht es, die Merkmale des Inspirations- und Exspirationszyklus sowie das Vorhandensein von Artefakten in einem bestimmten Zyklus zu bewerten und bei einer großen Anzahl von Artefakten eine Warnung vor möglichen Berechnungsfehlern auszugeben.

Bei der FRC-Bestimmung mittels Stickstoffauswaschung wird dem Patienten kurzzeitig eine erhöhte Sauerstoffkonzentration, beispielsweise 100 vol. %, angeboten oder ein Teil des Stickstoffs durch ein Edelgas ersetzt bei gleichbleibender Sauerstoffkonzentration. Für die Stickstoffauswaschung mit Hilfe einer erhöhten Sauerstoffkonzentration wird im Gegensatz zur Stickstoffauswaschung mit Hilfe von Edelgasen kein zusätzliches Gas bei einem Beatmungsgerät benötigt, weil Sauerstoff und normale Atemluft dort normalerweise zur Verfügung stehen.

für die FRC-Messung wird die Sauerstoffkonzentration im eingeatmeten Gas periodisch um einen vorgegebenen Wert verändert. Dafür ist ein Atemgasmischer vorgesehen, der dem Atemgas insbesondere 100% Sauerstoff zuführen kann und somit praktisch beliebige Sauerstoffkonzentrationen im Atemgas vorgeben kann. Der Atemgasmischer 20 kann die Sauerstoffkonzentration im eingeatmeten Gas erhöhen und verringern.

Die Sauerstoffkonzentration in der ausgeatmeten Luft ändert sich aufgrund der Trägheit der O2-Konzentrationsänderung in der funktionellen Residualkapazität gleichmäßig und mit einer Verzögerung. Das Gesetz der Konzentrationsänderung ist annähernd exponentiell.

Der Prozess der Stabilisierung der O2-Konzentration wird anhand der EtO2- und EtCO2-Werte am Ende der Ausatmung geschätzt.

Durch Messung der O2- und CO2-Menge in der ausgeatmeten Luft lässt sich auch eine Funktion der Stickstoffmenge in der ausgeatmeten Luft ermitteln:

Dies basiert darauf, dass zugemischter Sauerstoff den Stickstoff verdrängt. Bei 100 %iger O2-Beatmung wird der Stickstoff aus der Lunge verdrängt. In der Luft ist der Stickstoffanteil immer bei 79%. In Atemkreisläufen oder bei Atemgasen kann Sauerstoff den Stickstoff ersetzen. Basierend auf diesen Voraussetzungen kann der Stickstoffanteil berechnet werden.

$$N2(t) = Vexp(t) - O2(t) - CO2(t);$$

wobei:

- N2(t) - eine Funktion der Stickstoffmenge in der ausgeatmeten Luft;
- Vexp(t) - eine Funktion der Ausatmungsflussrate;
- O2(t) - eine Funktion der Sauerstoffmenge in der Ausatmungsluft;
- CO2(t) - eine Funktion der Kohlendioxidmenge in der Ausatmungsluft;

Es erfolgt eine Verwendung der Kurven für metabolische Berechnungen und die volumetrische Kapnometrie.

Der FRC-Berechnungsalgorithmus liefert das endexspiratorische Lungenvolumen:

$$FRC = (VN1-VN2)/(CN2-CN1)$$

wobei

VN1 - Inspiratorisches Stickstoffvolumen
VN2 - Exspiratorisches Stickstoffvolumen
CN1 - Stickstoffvolumenanteil in der vorhergehenden Ausatmung
CN2 - Anteil des Stickstoffvolumens bei der aktuellen Ausatmung

Die Berechnung wird beispielsweise bei jedem Atemzyklus nach der Änderung der Sauerstoffkonzentration durchgeführt. Es wird eine Mittelwertbildung über mehrere Zyklen durchgeführt.

**Patentansprüche**

1. Vorrichtung 14 zur zeitlich synchronisierten Messung von CO2- und O2-Anteilen im Atemgas und Atemgasfluss während der Beatmung

mit einer Inspirationsleitung 17, einer Exspirationsleitung 16 und einem Y-Verbinder 3 und einer Leitung zum Patientenanschluss 18, wobei der Y-Verbinder 3 die Inspirationsleitung 17, die Exspirationsleitung 16 und die Leitung 19 zum Patientenanschluss 18 verbindet, aufweisend einen Fluss-Sensor 2 zur Ermittlung von Strömungsdaten V(t), einen O2-Sensor 8 zur Ermittlung von O2(t) und einen CO2-Sensor 7 zur Ermittlung von CO2(t) und einen Prozessor 9 **dadurch gekennzeichnet, dass** der Prozessor 9 eingerichtet und ausgebildet ist,

- Strömungsdaten V(t) und CO2(t) im Speicher zu speichern,
- die Einatmungsphase IN oder die Ausatmungsphase EX im Verlauf von Messwerten des Strömungssensors 2 und der Sensoren für CO2 7 und für O2 8 zu bestimmen,
- den zeitlichen Versatz zwischen den Messwerten des Strömungssensors 2 und des CO2-Sensors 7 gegenüber dem O2-Sensor 8 zu bestimmen,
- die Messwerte entsprechend dem Zeitversatz zu synchronisieren.

2. Vorrichtung 14 nach Anspruch 1 **dadurch gekennzeichnet, dass** der Prozessor 9 eingerichtet und ausgebildet ist, die synchronisierten Konzentrationsfunktionen O2(t), CO2(t) und Fluss V(t) zur Berechnung der volumetrischen Größen VO2 und VCO2 zu verwenden.

3. Vorrichtung 14 nach einem der vorhergehenden Ansprüche **dadurch gekennzeichnet, dass** der Prozessor 9 eingerichtet und ausgebildet ist, die VO2- und VCO2-Daten zur Berechnung der Stoffwechselparameter REE und RQ unter Verwendung der Weir-Gleichung zu verwenden. Die REE- und RQ-Parameter werden für jeden Atemzyklus berechnet und über ein vom Benutzer gewähltes Zeitintervall gemittelt.

4. Vorrichtung 14 nach einem der vorhergehenden Ansprüche **dadurch gekennzeichnet, dass** der Prozessor 9 eingerichtet und ausgebildet ist, die V(t)- und VCO2(t)-Daten zur Erstellung eines volumetrischen Kapnometriediagramms zu verwenden, wobei V(t) auf der vertikalen und VCO2(t) auf der horizontalen Achse liegt.

5. Vorrichtung 14 nach einem der vorhergehenden Ansprüche **dadurch gekennzeichnet, dass** der Prozessor 9 eingerichtet und ausgebildet ist, die V(t)-, VO2(t)- und VCO2(t)-Daten zur Berechnung der funktionellen Rest-Lungenkapazität (FRC) zu verwenden.

6. Vorrichtung 14 nach einem der vorhergehenden Ansprüche **dadurch gekennzeichnet, dass** die Vorrichtung Teil eines Beatmungsgerätes 15 oder ein zusätzliches Modul für ein Beatmungsgerät ist, wobei das Beatmungsgerät 15 einen Sauerstoffmischer 20 aufweist.

7. Vorrichtung 14 nach einem der vorhergehenden Ansprüche **dadurch gekennzeichnet, dass** der Prozessor 9 ein Teil der Vorrichtung ist oder ein Teil des Beatmungsgerätes 15 ist.

8. Vorrichtung 14 nach einem der vorhergehenden Ansprüche **dadurch gekennzeichnet, dass** der Prozessor 9 eingerichtet und ausgebildet ist, für eine FRC-Messung die Sauerstoffkonzentration im eingeatmeten Atemgas mittels des Sauerstoffmischers 20 periodisch um einen vorgegebenen Wert verändern, und durch Messung der O2- und CO2-Menge in dem ausgeatmeten Atemgas eine Funktion der Stickstoffmenge in dem ausgeatmeten Atemgas zu ermitteln entsprechend der Formel

$$N2(t) = Vexp(t) - O2(t) - CO2(t);$$

wobei:

- N2(t) - eine Funktion der Stickstoffmenge in der ausgeatmeten Luft;
- Vexp(t) - eine Funktion der Ausatmungsflussrate;
- O2(t) - eine Funktion der Sauerstoffmenge in der Ausatmungsluft;
- CO2(t) - eine Funktion der Kohlendioxidmenge in der Ausatmungsluft.

9. Vorrichtung 14 nach einem der vorhergehenden Ansprüche **dadurch gekennzeichnet, dass** der Prozessor 9 eingerichtet und ausgebildet ist, die FRC entsprechend folgender Formel zu bestimmen:

$$FRC = (VN1-VN2)/(CN2-CN1)$$

wobei

- VN1 - Inspiratorisches Stickstoffvolumen
- VN2 - Exspiratorisches Stickstoffvolumen
- CN1 - Stickstoffvolumenanteil in der vorhergehenden Ausatmung
- CN2 - Anteil des Stickstoffvolumens bei der aktuellen Ausatmung

10. Vorrichtung 14 nach einem der vorhergehenden Ansprüche **dadurch gekennzeichnet, dass** der Prozessor 9 eingerichtet und ausgebildet ist, die Berechnungen bei jedem Atemzyklus nach der Änderung der Sauerstoffkonzentration durchzuführen.

11. Vorrichtung 14 nach einem der vorhergehenden Ansprüche **dadurch gekennzeichnet, dass** der O2-Sensor 8 und der CO2-Sensor 7 über eine Probenahmeleitung 13 mit dem Y-Verbindungsstück 3 verbunden sind, um Atemgas zu den Sensoren zu führen.

12. Vorrichtung 14 nach einem der vorhergehenden Ansprüche **dadurch gekennzeichnet, dass** die Vorrichtung eine Saugeinrichtung 6 aufweist, die eingerichtet ist Atemgas über die Probenahmeleitung 13 zu den Sensoren zu führen.

13. Vorrichtung 14 nach einem der vorhergehenden Ansprüche **dadurch gekennzeichnet, dass** der Fluss-Sensor 2 in einer Leitung zwischen dem Y-Verbinder 3 und dem Patienten 1 angeordnet ist.

14. Vorrichtung 14 nach einem der vorhergehenden Ansprüche **dadurch gekennzeichnet, dass** die Probenahmeleitung 13 auf der einen Seite mit dem Y-Verbindungsstück 3 und auf der anderen Seite über einen Probentrockner 12, welcher wässriges Kondensat aus dem analysierten Gas entfernt, verbunden ist und nachgeschaltet die Sensoren 7 und 8 angeordnet sind.

15. Verfahren zur zeitlich synchronisierten Messung von CO2- und O2-Anteilen im Atemgas und Atemgasfluss während der Beatmung

mit einer Inspirationsleitung 17, einer Exspirationsleitung 16 und einem Y-Verbinder 3 und einer Leitung zum Patientenanschluss 18, wobei der Y-Verbinder 3 die Inspirationsleitung 17, die Exspirationsleitung 16 und die Leitung 19 zum Patientenanschluss 18 verbindet, aufweisend einen Fluss-Sensor 2, einen O2-Sensor 8 und einen CO2-Sensor 7 und einen Prozessor 9 **dadurch gekennzeichnet, dass** der Prozessor 9 eingerichtet und ausgebildet ist, aus dem Verlauf der Messwerte von dem Fluss-Sensor 2, von dem O2-Sensor 8 und dem CO2-Sensor 7 die Atemphase Inspiration IN oder Exspiration EX zu ermitteln und aus der jeweiligen Atemphase einen Zeitversatz der Messwerte von dem Fluss-Sensor 2, von dem O2-Sensor 8 und dem CO2-Sensor 7 zueinander zu bestimmen und die Messwerte entsprechend dem Zeitversatz zu synchronisieren.

Fig. 1

Fig. 2

Fig. 3

Europäisches
Patentamt

European
Patent Office

Office européen
des brevets

# EUROPÄISCHER RECHERCHENBERICHT

Nummer der Anmeldung

EP 25 16 0571

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 2007/059263 A2 (CARDIOPULMONARY TECHNOLOGIES I [US]; RICCIARDELLI ROBERT H [US] ET AL.) 24. Mai 2007 (2007-05-24) | 1,4,6,7, 11-15 | INV. A61B5/083 A61B5/087 |
| Y | * Absatz [0002] - Absatz [0008] * * Absatz [0047] - Absatz [0048] * * Absatz [0054] - Absatz [0062] * * Absatz [0071] * * Absatz [0080] * * Absatz [0102] * ----- | 2,3,5, 8-10 | A61B5/091 A61B5/097 A61M16/08 A61M16/00 A61M16/10 |
| Y | EP 2 117 429 B1 (RIC INVESTMENTS LLC [US]) 10. Juni 2020 (2020-06-10) | 2,3 | |
| A | * Absatz [0025] * * Absatz [0126] * * Absatz [0120] * ----- | 6,7, 11-14 | |
| Y | US 2009/137919 A1 (BAR-LAVIE YARON [IL] ET AL) 28. Mai 2009 (2009-05-28) * Absatz [0044] - Absatz [0050] * ----- | 5,8-10 | |

RECHERCHIERTE SACHGEBIETE (IPC)

A61B
A61M

Der vorliegende Recherchenbericht wurde für alle Patentansprüche erstellt

| Recherchenort | Abschlußdatum der Recherche | Prüfer |
|---|---|---|
| Den Haag | 10. Juli 2025 | Gooding Arango, J |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

.......................................................

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**   EP 25 16 0571

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

10-07-2025

| Im Recherchenbericht angeführtes Patentdokument | Datum der Veröffentlichung | Mitglied(er) der Patentfamilie | Datum der Veröffentlichung |
|---|---|---|---|
| WO 2007059263 A2 | 24-05-2007 | CN 101547716 A | 30-09-2009 |
|  |  | EP 1951347 A2 | 06-08-2008 |
|  |  | US 2007107728 A1 | 17-05-2007 |
|  |  | WO 2007059263 A2 | 24-05-2007 |
| EP 2117429 B1 | 10-06-2020 | BR PI0807019 A2 | 16-06-2015 |
|  |  | CN 101600391 A | 09-12-2009 |
|  |  | EP 2117429 A2 | 18-11-2009 |
|  |  | JP 6005330 B2 | 12-10-2016 |
|  |  | JP 2010517635 A | 27-05-2010 |
|  |  | RU 2009132671 A | 10-03-2011 |
|  |  | US 2007225612 A1 | 27-09-2007 |
|  |  | WO 2008095120 A2 | 07-08-2008 |
| US 2009137919 A1 | 28-05-2009 | AU 2006286163 A1 | 08-03-2007 |
|  |  | EP 1919353 A2 | 14-05-2008 |
|  |  | JP 2009506825 A | 19-02-2009 |
|  |  | US 2009137919 A1 | 28-05-2009 |
|  |  | WO 2007026367 A2 | 08-03-2007 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5540233 A
- US 2022257141 A1
- US 2002052560 A1
- US 8371298 B2